# EUROPEAN PATENT APPLICATION

(11) **EP 3 904 530 A1**
(43) Date of publication of application: **03.11.2021**
(21) Application number: 20171814.5
(22) Date of filing: 28.04.2020
(51) Int. Cl.: C12Q 1/6806, C12Q 1/6841, C12Q 1/689, C12Q 1/6895

(54) **METHOD FOR COLLECTION, DETECTION AND/OR IDENTIFICATION OF MICROORGANISMS FROM A NATURAL STONE AND USES THEREOF**

(30) Priority: 27.04.2020 PT 2020020726
(71) Applicant: Universidade de Évora, 7000-803 Évora (PT)
(72) Inventor: Fialho Caeiro Caldeira, Ana Teresa, 7000-651 Évora (PT); Oliveira Vieira, Ricardo Nuno, 4755-402 Barcelos (PT); Macedo Arantes, Sílvia Alexandra, 7000-582 Évora (PT); González Pérez, Marina, 2615-022 Alverca do Ribatejo, Vila Franca de Xira (PT); Estevão Grande Candeias, António José, 7000-651 Évora (PT); Paulo Mirão, José António, 7000-019 Nª Sr.da Graça do Divor (PT)
(74) Representative: Patentree

(57) **Abstract**

The present disclosure relates to the use of a nylon membrane as a carrier for collection, detection and/or identification of microorganisms from a natural stone, wherein the detection and/or identification is performed using fluorescent *in situ* hybridisation techniques. The disclosure also describes a method to collect and identify microorganisms at the nylon membrane. A kit and an article comprising a nylon membrane for use as carrier for collection and identification of microorganisms from a natural stone, are also encompassed.

## Description

### TECHNICAL FIELD

The present disclosure relates to a non-destructive method for detection of microbial cells in marble stone. The method allows the detection of biologically active microorganisms by fluorescence *in situ* hybridisation (FISH), using a nylon membrane for sampling and detection of the biologically active microorganisms.

### BACKGROUND

Biodeterioration is an undesirable process, triggered by living organisms, which can affect cultural and built heritage and economically important materials, such as marble monuments. This is a relevant issue in the conservation of monuments and cultural artefacts that represent a widely diversified group of ecological niches for various microorganisms. Although several biotic and abiotic conditions such as humidity, temperature, luminosity and chemical factors like the nature of the substratum can accelerate this process, microorganisms are perhaps its main promoters [1].

The development of new techniques and methodologies to easily detect and identify the microorganisms colonizing cultural heritage and to determine the microbial community involved in its deterioration is crucial to design appropriated effective treatment or preventive strategies.

Detection and identification of microbial communities that are present in cultural heritage materials can be performed through the use of different complementary methods, and new approaches are continuously being developed. In the near past, research carried out in this field was based mainly on classical culture-based methodologies. However, these methodologies are time consuming and omit slow growing and uncultivable microorganisms that may account for more than 90% of the microflora present [2,3].

To overcome this drawback, culture-independent techniques based in molecular approaches, that are more sensitive and need smaller quantities of sample than those previously mentioned, are being introduced to study microorganisms [4,5]. These include Random Amplified Polymorphic DNA (RAPD), MicroSatelite Primer-Polimeric Chain Reaction (MSP-PCR), restriction fragment length polymorphism (RFLP), Denaturing Gradient Gel Electrophoresis (DGGE) and Next GenerationSequencing (NGS).

However, all these methods are difficult to implement, expensive, require expert knowledge and have an important limitation: the impossibility to study the microorganisms *in situ* or at point-of-care.

The fluorescent *in situ* hybridisation (FISH) [6,7] is a simple, rapid and promising molecular cytogenetic technique enabling for detection, visualisation and identification of the microorganisms of interest in materials of cultural heritage. This methodology devised an alternative way for fast screening and identification of target microorganisms by applying a "non-PCR"-based technique that combines the precision of molecular techniques and provides information on the number and spatial distribution of metabolic active microorganisms. FISH allows to detect microorganisms by a fluorescently labelled oligonucleotide target probe that hybridizes specifically to its complementary target sequence within the cell [15]. The selection of ribosomal ribonucleic acid (rRNA) probes enables phylogenetic specificity to be varied from the universal to the subspecies level [14].

Surprisingly, only few studies have explored the application of FISH, particularly FISH to detect and localize specific RNA targets (RNA-FISH), in the field of cultural heritage conservation and restoration [8-11] even though it is one of the most common techniques applied among the "non-PCR"-based techniques in other fields [2,3,14].

These facts are disclosed in order to illustrate the technical problem addressed by the present disclosure.

### GENERAL DESCRIPTION

The present disclosure relates to the use of a nylon membrane as a carrier for collection, detection and/or identification of a microorganisms from a natural stone, preferably limestone, more preferably marble.

In an embodiment, biofilms can be sampled from marble specimens with a nylon membrane, that is then used as physical support for the direct detection of microorganism by RNA-FISH. The combination of non-destructive sampling and RNA-FISH detection simplifies the identification process, making it more efficient, cheaper, and safer (since it uses fewer toxic components), giving the possibility to be carried out by non-specialists.

Nylon membranes are hydrophilic yet solvent-resistant, and therefore widely used for filtering aqueous solutions as well as organic solvents. Moreover, these membranes are strong and flexible.

In an embodiment, the nylon membrane has a pore size less or equal to 0.2 µm.

In an embodiment, the identification of microorganisms is performed by molecular cytogenetic techniques. In a further embodiment, the molecular cytogenetic technique is fluorescent *in situ* hybridisation (FISH).

In an embodiment, the fixation, permeabilization, hybridization, washing and detection steps of the fluorescent *in situ* hybridisation protocol are performed directly on the nylon membrane.

In an embodiment, this disclosure relates to the use of a nylon membrane for collection of microorganisms, wherein the collection is non-destructive.

The present disclosure also relates to a kit for collection, detection and/or identification of microorganisms comprising a nylon membrane for use as described in other embodiments of the present disclosure.

An aspect of the present disclosure relates to an article comprising a nylon membrane for use as described in the embodiments of this disclosure.

The present document also discloses a method for collecting, detecting and/or identifying microorganisms from a natural stone, comprising the following steps: (i) impregnating with sterile water a nylon membrane, wherein the nylon membrane has a pore size less or equal to 0.2 µm; (ii) collecting the microorganisms by pressing the nylon membrane on the natural stone; (iii) mounting the nylon membrane in a filter holder, preferably on a polypropylene filter holder; (iv) filling the filter holder with sterile water; (v) fixing and permeabilizing the collected microorganism cells using microwaves, preferably at a power of 130 W for at least 30 seconds; (vi) covering the nylon membrane with a solution comprising hybridisation buffer and fluorescent probes suitable for fluorescent *in situ* hybridisation; (vii) incubating the filter holder and the nylon membrane at 40-50°C, preferably at 46°C for 2 hours; (viii) washing the nylon membrane with sterile water; (ix) covering the nylon membrane with hybridisation buffer; (x) incubating the filter holder and the nylon membrane at 40-50°C, preferably at 46°C for 30 minutes; (xi) washing with sterile water; (xii) retrieving the nylon membrane from the filter holder; and (xiii) observing the nylon membrane under a fluorescence microscope for detecting and/or identifying a microorganism from a natural stone.

In an embodiment wherein the fixation and permeabilization of the collected microorganism cells is obtained by using microwaves, formaldehyde, or ethanol, preferably using microwaves at a power of 130 W for at least 30 seconds.

In an embodiment, the filter holder is a polypropylene filter holder.

In an embodiment, the filter holder and the nylon membrane are incubated at 40-50°C, preferably at 46°C for 2 hours.

### BRIEF DESCRIPTION OF THE DRAWINGS

The following figures provide preferred embodiments for illustrating the disclosure and should not be seen as limiting the scope of invention.

The present disclosure is best understood from the following detailed description when read in connection with the accompanying drawing. It is emphasized that, according to common practice, the various features of the drawing are not to scale. On the contrary, the dimensions of the various features are arbitrarily expanded or reduced for clarity.
**Figure 1** shows a representation graphic of the relative recovery index for different types of sampling in marble specimens.
**Figure 2** shows the fluorescent signs of microorganisms after applying the RNA-FISH technique in the nylon membrane for *Rhodotorula* (a) *Bacillus* (b) and *Penicillium* spores (c) in marble assays.
**Figure 3** is a schematic representation of the filter holder used to mount the nylon membrane.
**Figure 4** shows the fluorescent signs of bacteria present in marble monument, directly observed on the nylon membrane.

### DETAILED DESCRIPTION

The present disclosure relates to the use of a nylon membrane as a carrier for collection, detection and/or identification of microorganisms from a natural stone, wherein the detection and/or identification is performed using fluorescent in situ hybridisation techniques. The disclosure also describes a method to collect and identify microorganisms at the nylon membrane. A kit and an article comprising a nylon membrane for use as carrier for collection and identification of microorganisms from a natural stone, are also encompassed.

The proposed methodology combines a non-destructive sampling method with nylon membranes with detection, visualization and identification of organisms by RNA-FISH. The entire process for the detection of microbial communities (fixation; permeabilization; hybridization; washing and detection) was adapted and optimized to be performed directly on the nylon sampling membrane. That allows a very simplified procedure for non-destructive tests, effective in detecting metabolically active microbial communities in marble samples.

This methodology allows the sampling and detection of bacteria, yeasts and filamentous fungi, and can be applied with universal probes or with other probes aimed at specific taxonomic groups within microbial communities. These probes should be suitable for FISH and marked with the fluorophore CY3 in the 5 'position, or other fluorophores, also in the 5' position of the probe.

The present disclosure relates to the use of a nylon membrane carrier for sampling and detection of microorganisms collected from natural stone.

In an embodiment, three different biological models were used to artificially inoculate marble samples: yeasts of the genus *Rhodotorula,* bacteria of the genus *Bacillus* and filamentous fungi of the genus *Penicillium,* belonging to the collection of cultures of unit 2BU (Biotechnology and Biodegradation Unit, Lab HERCULES, University of Évora). Unknown microorganism samples were collected from an historical monument built in Marble (convent of Maltese, Estremoz, Portugal).

In an embodiment, marble specimens were prepared and artificially inoculated to mimic contaminated cultural heritage materials. Sterile specimens (1x1 cm) were inoculated with 1.5x10⁸ yeast and bacteria cells in exponential growth phase and 1x10⁸ filamentous fungus spores (resuspended in miliQ water). These replicas were incubated for 2 weeks at 28°C.

In an embodiment, the inoculated marble specimens were used to optimize the sampling process and subsequent detection of microorganisms by RNA-FISH. The biofilms from each marble specimen were sampled with 4 different materials, using non-destructive methodologies: i) swabbing with cotton swab for 30 seconds in the stone surface; ii) pressing 1 cm² of Whatman® filter paper no. 2 against the stone surface; iii) pressing of 1 cm² nylon membrane impregnated with sterile distilled water for 30 seconds in the stone surface; iv) pressing of 1 cm² nitrocellulose membrane impregnated with sterile distilled water for 30 seconds in the stone surface. After the 30 seconds of sampling, the material was transferred to a sterile microtube to carry out cell fixation and permeabilization.

In an embodiment, yeast cells and bacteria recovered from each one of the materials, i.e. cotton swabs, filter paper, nitrocellulose membrane and nylon membrane, were fixed by adding 1 mL of absolute ethanol to each tube, respectively. Filamentous fungi were fixed with 1 mL paraformaldehyde at 4% (v/v). The fixation step occurred for 1 hour at 25°C. Alternatively, samples can be microwaved for 30 seconds, using medium-high power. In the end, 1 mL of phosphate buffered saline (PBS, 130.0 mM NaCl, 8.0 mM NaH₂PO₄, 2.7 mM KCI, 1.5 mM KH₂PO₄, pH 7.2) was added.

The obtained cell suspensions were used to i) determine cell recovery capacity using each of the selected sampling methods (per count in the Neubauer chamber) and ii) evaluate the performance of the RNA-FISH technique associated with the different sampling methods to detect the target microorganisms.

In an embodiment, for the hybridization with fluorescent probes the samples were stirred in vortex for 30 seconds and subjected to orbital agitation at 100 rpm at 30°C for 12 hours. After this period, the materials used for sampling were discarded and the tubes were centrifugated at 15 000g for 20 minutes. The resulting cellular sediment was resuspended with 80 µl of hybridization buffer (HB, 0.9 M NaCl, 20 mM Tris-HCI (pH 7.2), 0.1% (m/v) sodium dodecyl sulfate) was added. The obtained suspension was separated into three microtubes to perform the hybridization tests.

In a further embodiment, RNA-FISH assays were performed in triplicate using:
- One sample, with two individual Probes EUK516-Cy3 or EUB338-Cy3(universal probes for bacteria domain, EUB338-Cy3 (SEQ. ID. 1 - 5'Cy3-GCTGCCTCCCGTAGGAGT-3), and eukarya domain, EUK516-Cy3 ((SEQ: ID. 2 - 5'Cy3-ACCAGACTTGCCCTCC-3);
- One negative control (NONEUB-Cy3);
- One blank (no probe).

In an embodiment, in the tests in which probes were added, 0.25 µl of each probe (120 ng/µL) were added. Hybridization took place under agitation for 2 hours at 46°C in a water bath. Subsequently, the samples were centrifuged at 15 000g for 20 minutes, and the precipitate was suspended in 25 µL of pre-heated hybridization buffer for 30 minutes at 46°C. Cells were then recovered by centrifugation (15 000g for 20 minutes) and re-suspended in ultrapure water.

In an embodiment, microorganisms were detected after the hybridization process by fluorescence detection, performed using an Epifluorescence microscope with mercury lamp (100W).

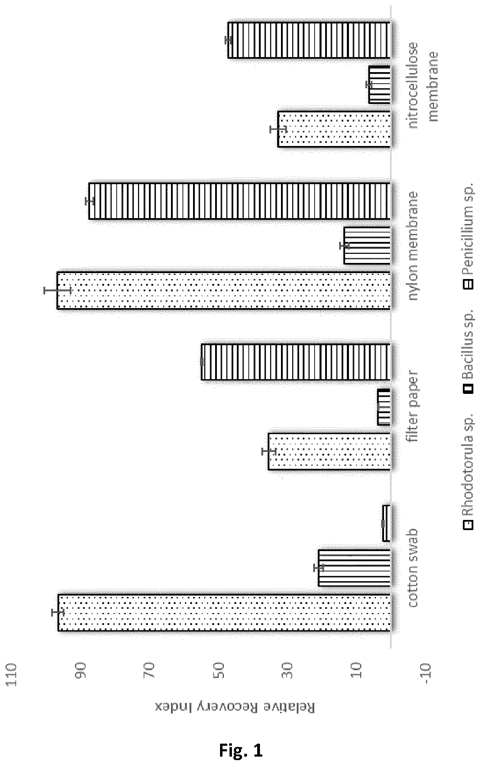

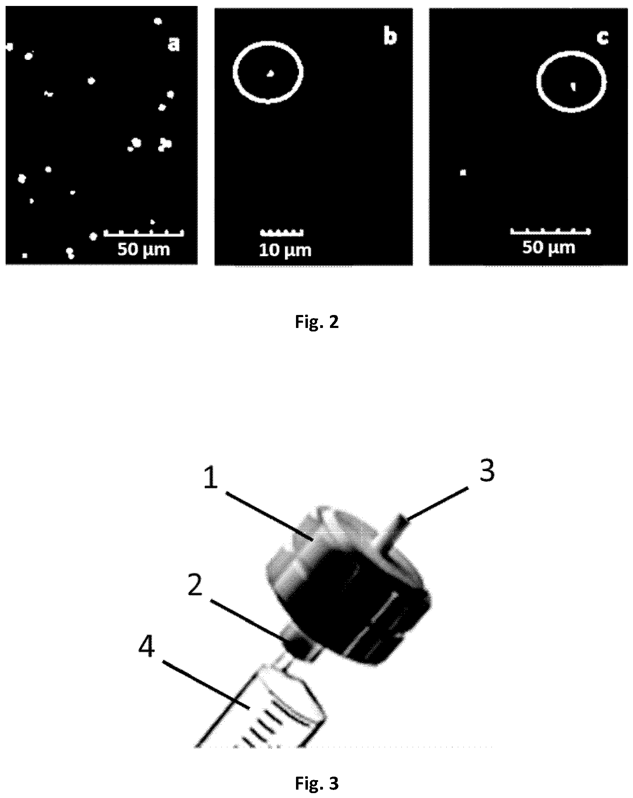

The Relative Recovery Index (RI) obtained for the different materials used on the sampling step of marble specimens is plotted in Fig. 1. The nylon membrane sampling method allowed a higher RI of yeasts and filamentous fungal spores and cotton swab for bacteria (Fig. 1). Regardless, the nylon membranes allowed to recover simultaneously with high Relative Recovery Index bacteria, yeasts and filamentous fungi from marble surfaces. Samples collected using this material were successfully identified using the RNA-FISH technique. As depicted in Fig. 2, good fluorescent signals were found after RNA-FISH for the microorganisms inoculated in the marble specimens (yeasts, bacteria and filamentous fungi), showing that RNA-FISH can be combined with non-destructive sampling for detection/identification of active microorganisms in marble samples.

In another embodiment, assays were carried out in a historical monument built in marble, the convent of Maltezas (Estremoz, Portugal). Active microbial communities in marble stone were collected using a nylon membrane and the RNA-FISH protocol was performed directly in the nylon membranes. The detection step was performed by direct observation of the processed nylon membrane using fluorescence microscopy.

In an embodiment, the nylon membrane, with a diameter of 13 mm and pore size of 0.2 µm, was impregnated with 100 µL of sterile distilled water. The membrane was then pressed against a marble surface, for at least 30 seconds, to collect the microbial sample. After collection, the nylon membrane was placed inside a polypropylene syringe filter holder 1, suitable for the size of the membrane used.

In an embodiment, after sample collection the nylon membrane was washed using sterile water, preferably using 100 µL of sterile MiliQ water. Then, cells were fixed and permeabilized, using microwaves. For that, the filter holder, with the nylon membrane, was microwaved for 30 seconds at 130 W.

In a further embodiment, the RNA-FISH probe was prepared in a microtube using 1 µl of probe at 120 ng/µl and HB buffer, making up a final volume of 200 µL. The RNA-FISH probe solution was then inserted inside the filter holder, by pipetting into the top hole **2.** To retain this solution inside the filter holder, the two apertures of the holder **2,3** were tightly covered.

In an embodiment, the nylon membrane, placed inside the filter holder system, was incubated with the RNA-FISH probe solution at 46°C for 2 hours. After that, the top hole of the filter holder was opened to insert a syringe **4,** previously filled with 4 mL of sterilized MiliQ water. The water in the syringe was then used to wash the membrane, by pressing the syringe plunger, thus forcing the water to enter in the filter holder, and consequently pass through the nylon membrane. After the washing step, the syringe was dismounted from the filter holder. The filter holder was then filled with HB, preferably 200 µL of HB, the top hole was closed, and the system incubated for 30 minutes at 46°C. After the incubation with HB, the membrane was washed again by passing sterilized MiliQ water through the filter holder, using a syringe.

In an embodiment, the filter holder was dismounted after the second wash with sterile water, and the nylon membrane retrieved using forceps. The retrieved membrane was then placed on the top of microscope slide, covered with a coverslip and fixed using adhesive tape.

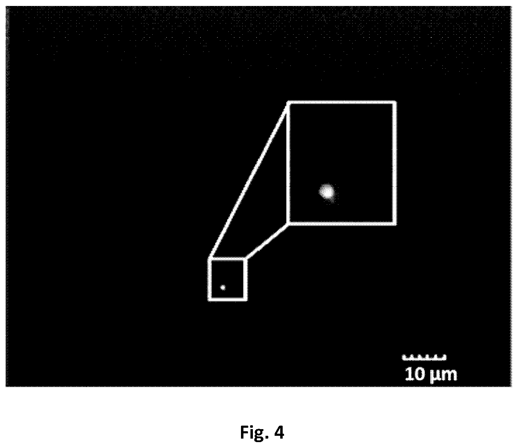

In a further embodiment, the nylon membrane mounted in the microscope slide was observed on an epifluorescence microscope. As depicted in Fig. 4, it was possible to identify bacteria directly on the nylon membrane.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

### REFERENCES

[1] Rojas et al., International Biodeterioration & Biodegradation 2009, 63, 169-175.
[2] Amann et al., Current Opinion in Biotechnology 2001, 12, 231-236.
[3] Moter and Göbel, Journal of Microbiological Methods 2000, 41, 85-112.
[4] Rosado et al., International Biodeterioration & Biodegradation 2013, 85, 1-7.
[5] Rosado et al., Analytical and Bioanalytical Chemistry, 2014, 406, 887-895.
[6] Bottari et al, E. Appl. Microbiol. Biotechnol. 2006, 73, 485-94.
[7] Aoi, Journal of Bioscience and Bioengineering 2002, 94, 552-556.
[8] Cappitelli and Sorlini, Applied and Environmental Microbiology 2008, 74, 564-569.
[9] Urzì et al., Environmental Microbiology 2004, 6, 678-85.
[10] Vieira R et al. Conserv. Património, vol. 23, pp. 71-77, 2016.
[11] Gonzalez-Perez et al Appl. Phys. A, vol. 123, no. 2, p. 142, 2017.
[12] La Cono and Urzi, Journal of Microbiological Methods 2003, 55, 65-71.
[13] Urzì and De Leo, Journal of Microbiological Methods 2001, 44, 1-11.
[14] Amann et al., Applied and Environmental Microbiology 1990, 56, 1919-1925.
[15] Raafat et al, Appl. Environ. Microbiol., 2008, 74, 3764-3773.

## Claims

1. Use of a nylon membrane as a carrier for collection, detection and/or identification of a microorganisms from a natural stone, preferably limestone, more preferably marble.

2. Use of a nylon membrane according to any the previous claims wherein the nylon membrane has a pore size less or equal to 0.2°µm.

3. Use of a nylon membrane according to the previous claim wherein the identification of microorganisms is performed by molecular cytogenetic techniques.

4. Use of a nylon membrane according to any the previous claims wherein the molecular cytogenetic technique is fluorescent *in situ* hybridisation.

5. Use of a nylon membrane according to any the previous claims wherein the fixation, permeabilization, hybridization, washing and detection steps of the fluorescent *in situ* hybridisation protocol are performed directly on the nylon membrane.

6. Use of a nylon membrane according to any the previous claims wherein the collection of microorganisms is non-destructive.

7. Kit for collection, detection and identification of microorganisms comprising a nylon membrane for use as described in any of the previous claims.

8. Article comprising a nylon membrane for use as described in any of the previous claims.

9. Method for collecting, detecting and/or identifying microorganisms from a natural stone, comprising the following steps:
impregnating with water a nylon membrane, wherein the nylon membrane has a pore size less or equal to 0.2 µm;
collecting the microorganisms by pressing the nylon membrane on the natural stone;
mounting the nylon membrane in a filter holder;
filling the filter holder with sterile water;
fixing and permeabilizing the collected microorganism cells;
covering the nylon membrane with a solution comprising hybridisation buffer and fluorescent probes suitable for fluorescent *in situ* hybridisation;
incubating the filter holder and the nylon membrane;
washing the nylon membrane;
covering the nylon membrane with hybridisation buffer;
incubating the filter holder and the nylon membrane;
washing the nylon membrane;
retrieving the nylon membrane from the filter holder; and
observing the nylon membrane under a fluorescence microscope for detecting and/or identifying a microorganism from a natural stone.

10. Method according to the previous claim wherein the fixing and permeabilizing the collected microorganism cells is obtained by using microwaves, formaldehyde, or ethanol, preferably using microwaves at a power of 130 W for at least 30 seconds.

11. Method according to any of the previous claims 9-10 wherein the filter holder is a polypropylene filter holder.

12. Method according to any of the previous claims 9-11 wherein the filter holder and the nylon membrane are incubated at 40-50°C, preferably at 46°C for 2 hours.

13. Method according to any of the previous claims 9-12 wherein the water is sterile water.


SEQUENCE LISTING

<110> Universidade de Evora

<120> METHOD FOR COLLECTION, DETECTION AND/OR IDENTIFICATION OF
MICROORGANISMS FROM A NATURAL STONE AND USES THEREOF

<130> P901.1 EP

<160> 2

<170> BiSSAP 1.3.6

<210> 1
<211> 18
<212> DNA
<213> Artificial Sequence

<220>
<223> EUB338-Cy3

<400> 1
gctgcctccc gtaggagt                                                    18

<210> 2
<211> 16
<212> DNA
<213> Artificial Sequence

<220>
<223> EUK516-Cy3

<400> 2
accagacttg ccctcc                                                      16